(19) **Europäisches Patentamt / European Patent Office / Office européen des brevets**

(11) **EP 2 735 271 A1**

(12) # EUROPEAN PATENT APPLICATION

(43) Date of publication:
28.05.2014 Bulletin 2014/22

(51) Int Cl.:
*A61B 8/08* (2006.01)   *A61B 8/00* (2006.01)
*A61B 19/00* (2006.01)   *G06T 7/00* (2006.01)

(21) Application number: 13191054.9

(22) Date of filing: 31.10.2013

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 23.11.2012 KR 20120134005

(71) Applicant: Samsung Medison Co., Ltd.
Gangwon-do 250-870 (KR)

(72) Inventors:
• **Lee, Jae-keun**
  **Gangwon-do (KR)**
• **Baek, Ji-hye**
  **Gangwon-do (KR)**

(74) Representative: **Lorenz, Markus**
**Lorenz & Kollegen**
**Patentanwälte Partnerschaftsgesellschaft**
**Alte Ulmer Straße 2**
**89522 Heidenheim (DE)**

(54) **Ultrasound system and method for providing guideline of needle**

(57)   Disclosed are an ultrasound system and method for providing a guideline of a needle. The ultrasound system includes an ultrasound data acquirer that transmits an ultrasound signal to an object with a needle inserted thereinto, and acquires ultrasound data corresponding to each of a plurality of ultrasound images by receiving an ultrasound echo signal reflected from the object, a user input unit that receives input information, used to set a point in an ultrasound image, from a user, and a processor that generates the plurality of ultrasound images with the ultrasound data, generates a mask image used to detect an input position of the needle by using the plurality of ultrasound images, detects the input position by using the mask image, and sets a guideline of the needle in the ultrasound image on a basis of the input position and the input information.

FIG. 1

EP 2 735 271 A1

**Description**

RELATED APPLICATIONS

This application claims the benefit of Korean Patent Application No. 10-2012-0134005, filed on November 23, 2012, in the Korean Intellectual Property Office, the disclosure of which is incorporated herein in its entirety by reference.

BACKGROUND

1. Field

One or more embodiments of the present invention relate to an ultrasound system, and more particularly, to an ultrasound system and method for providing a guideline of a needle.

2. Description of the Related Art

With the advance of medical technology, technology is being used in which a user forms a minimum-size hole in an object without directly incising the object, inserts a medical needle, such as an ablator or a biopsy, into a body part having a lesion while looking at an internal image of the object, and treats or examines the lesion. Such a method performs a medical procedure while observing the inside of an object by using a medical image apparatus such as a computed tomography (CT) apparatus or a magnetic resonance imaging (MRI) apparatus, and thus is called a procedure therapy using an image or an interventional procedure therapy. That is, an interventional procedure denotes a medical procedure in which an operator directly brings a medical needle into contact with a lesion requiring an examination or a procedure while looking at an image obtained from an MRI apparatus or a CT apparatus during a medical procedure, and treats or examines the lesion. In comparison with a surgical treatment, the interventional procedure therapy does not require general anesthesia, is low in physical burden of an object, is low in pain, shortens a hospitalization period, and enables a patient to return to daily life early after a medical procedure, thus providing many benefits in terms of medical cost and effect.

However, when using a CT apparatus or an MRI apparatus, it is difficult to obtain an image of an object in real time. Also, when performing an interventional procedure with a CT apparatus, an operator and an object have a risk of being exposed to radioactivity for a long time. On the other hand, when using an ultrasound system, an ultrasound image is obtained in real time, and moreover, it is almost harmless to an object.

SUMMARY

One or more embodiments of the present invention include an ultrasound system and method that detect an input position of a needle by using an ultrasound image, and sets a guideline of the needle on the basis of the input position of the needle and a point which is set on the ultrasound image by a user. (see Claims for changes to the remaining Summary)

Additional aspects will be set forth in part in the description which follows and, in part, will be apparent from the description, or may be learned by practice of the presented embodiments.

According to one or more embodiments of the present invention, an ultrasound system includes: an ultrasound data acquirer that transmits an ultrasound signal to an object with a needle inserted thereinto, and acquires ultrasound data corresponding to each of a plurality of ultrasound images by receiving an ultrasound echo signal reflected from the object; a user input unit that receives input information, used to set a point in an ultrasound image, from a user; and a processor that generates the plurality of ultrasound images with the ultrasound data, generates a mask image used to detect an input position of the needle by using the plurality of ultrasound images, detects the input position by using the mask image, and sets a guideline of the needle in the ultrasound image on a basis of the input position and the input information.

The processor may generate a plurality of copy ultrasound images by down-sampling the plurality of ultrasound images.

The processor may generate a motion image of the needle by using a certain number of copy ultrasound images among the plurality of copy ultrasound images.

The processor may select a certain number of copy ultrasound images in a temporal direction with respect to each of the plurality of copy ultrasound images.

The processor may generate the motion image of the needle with the following Equation (1):

$$NMI_i = \frac{|CUI_{i-2}-CUI_{i-1}|+|CUI_{i-1}-CUI_i|+|CUI_i-CUI_{i+1}|+|CUI_{i+1}-CUI_{i+2}|}{4} \qquad \cdots(1)$$

where $NMI_i$ denotes a needle motion image, and $CUI_{i-2}$ to $CUI_{i+2}$ respectively denote the selected copy ultrasound images.

The processor may generate a mask image used to emphasize the needle in the ultrasound image by using the ultrasound image and the motion image.

The processor may generate the mask image with the following Equation (2):

$$MI_i = UI_i \times \alpha + NMI_i \times (1-\alpha) \qquad \cdots(2)$$

where MI denotes the mask image, UI denotes the ultrasound image, and $\alpha$ denotes a weight value.

The processor may set a region of interest (ROI) having a predetermined size in the mask image in consideration of an input direction of the needle, calculate a plurality of brightness accumulation values by accumulating brightness values of respective pixels having different depths of the mask image in a width direction of the mask image, for a plurality of pixels in the ROI, detect a maximum brightness accumulation value from the plurality of the calculated brightness accumulation values, and detects a depth, corresponding to the detected maximum brightness accumulation value, as the input position of the needle.

The processor may set the input position and the point in the ultrasound image based on the input position of the needle and the input information, and set a line, which connects the input position and the point, as the guideline.

The processor may detect a motion of the point by performing motion tracking between adjacent ultrasound images with respect to the point set on each of the plurality of ultrasound images.

The processor may measure a distance between the point and the input position.

The processor may calculate an angle of the guideline with respect to the input position.

The processor may detect an input angle of the needle on a basis of the input position.

The processor may calculate a first average value of the plurality of brightness accumulation values, calculates a second average value of brightness accumulation values which are obtained by subtracting the first average value from the plurality of the brightness accumulation values, detects an intersection point between the second average value and the plurality of brightness accumulation values as a start point and an end point, calculates a length between the detected start point and end point, and calculates the input angle of the needle based on the calculated length.

The processor may calculate the input angle of the needle with the following Equation (3):

$$\theta_{IA} = \tan^{-1}\left(\frac{nNDLength}{\frac{W}{5}}\right) \qquad \cdots(3)$$

where $\Theta$ denotes the input angle of the needle, nNDLength denotes the length, and W denotes a width of the mask image.

The processor may set a plurality of angles with respect to the input position of the needle, set a line corresponding to each of the plurality of angles from the input position of the needle, calculate brightness accumulation values of pixels of a line corresponding to each of the plurality of angles, calculates a maximum brightness accumulation value from the calculated brightness accumulation values, and detect an angle, corresponding to the detected maximum brightness accumulation value, as the input angle of the needle.

According to one or more embodiments of the present invention, a method of providing a guideline of a needle includes: a) transmitting an ultrasound signal to an object with a needle inserted thereinto, and acquires ultrasound data corresponding to each of a plurality of ultrasound images by receiving an ultrasound echo signal reflected from the object; b) generating the plurality of ultrasound images with the ultrasound data; c) receiving input information, used to set a point in an ultrasound image, from a user; d) generating a mask image used to detect an input position of the needle by using the plurality of ultrasound images; e) detecting the input position by using the mask image; and f) setting a guideline of the needle in the ultrasound image based on the input position and the input information.

Operation d) may include d1) generating a plurality of copy ultrasound images by down-sampling the plurality of ultrasound images.

Operation d) may include d2) generating a motion image of the needle by using a certain number of copy ultrasound images among the plurality of copy ultrasound images.

Operation d2) may includes selecting a certain number of copy ultrasound images in a temporal direction with respect to each of the plurality of copy ultrasound images.

The generating of a motion image may include generating the motion image of the needle with the following Equation (1):

$$NMI_i = \frac{|CUI_{i-2}-CUI_{i-1}|+|CUI_{i-1}-CUI_i|+|CUI_i-CUI_{i+1}|+|CUI_{i+1}-CUI_{i+2}|}{4} \quad \cdots (1)$$

where $NMI_i$ denotes a needle motion image, and $CUI_{i-2}$ to $CUI_{i+2}$ respectively denote the selected copy ultrasound images.

Operation d) may include d3) generating the mask image used to emphasize the needle in the ultrasound image by using the ultrasound image and the motion image.

Operation d3) may include generating the mask image with the following Equation (2):

$$MI_i = UI_i \times \alpha + NMI_i \times (1-\alpha) \quad \cdots (2)$$

where MI denotes the mask image, UI denotes the ultrasound image, and $\alpha$ denotes a weight value.

Operation e) may include: setting a region of interest (ROI) having a predetermined size in the mask image in consideration of an input direction of the needle; calculating a plurality of brightness accumulation values by accumulating brightness values of respective pixels having different depths of the mask image in a width direction of the mask image, for a plurality of pixels in the ROI; detecting a maximum brightness accumulation value from the plurality of the calculated brightness accumulation values; and detecting a depth, corresponding to the detected maximum brightness accumulation value, as the input position of the needle.

Operation f) may include setting the input position and the point in the ultrasound image based on the input position of the needle and the input information, and setting a line, which connects the input position and the point, as the guideline.

Operation f) may include detecting a motion of the point by performing motion tracking between adjacent ultrasound images with respect to the point set on each of the plurality of ultrasound images.

The method may further include g) measuring a distance between the point and the input position.

Operation g) may include detecting an angle of the guideline with respect to the input position.

Operation g) may include detecting an input angle of the needle on a basis of the input position.

Operation g) may include: calculating a first average value of the plurality of brightness accumulation values; calculating a second average value of brightness accumulation values which are obtained by subtracting the first average value from the plurality of brightness accumulation values; detecting an intersection point between the second average value and the plurality of brightness accumulation values as a start point and an end point; calculating a length between the detected start point and end point; and calculating the input angle of the needle based on the calculated length.

The calculating of an input angle may include calculating the input angle of the needle with the following Equation (3):

$$\theta_{IA} = \tan^{-1}\left(\frac{nNDLength}{\frac{W}{5}}\right) \quad \cdots (3)$$

where $\Theta$ denotes the input angle of the needle, nNDLength denotes the length, and W denotes a width of the mask image.

Operation g) may include: setting a plurality of angles with respect to the input position of the needle; setting a line corresponding to each of the plurality of angles from the input position of the needle; calculating brightness accumulation values of pixels of a line corresponding to each of the plurality of angles; calculating a maximum brightness accumulation value from the calculated brightness accumulation values; and detecting an angle, corresponding to the detected maximum brightness accumulation value, as the input angle of the needle.

BRIEF DESCRIPTION OF THE DRAWINGS

These and/or other aspects will become apparent and more readily appreciated from the following description of the embodiments, taken in conjunction with the accompanying drawings in which:

FIG. 1 is a block diagram of a configuration of an ultrasound system according to an embodiment of the present invention;

FIG. 2 is a block diagram of a configuration of an ultrasound data acquirer according to an embodiment of the present invention;

FIG. 3 is an exemplary diagram illustrating a plurality of ultrasound images with time;

FIG. 4 is a flowchart of an operation of setting a guideline of a needle, according to an embodiment of the present invention;

FIG. 5 is an exemplary diagram illustrating a mask region and a first region of interest (ROI) according to an embodiment of the present invention;

FIG. 6 is an exemplary diagram illustrating a needle input position and a brightness accumulation value based on a depth, according to an embodiment of the present invention;

FIG. 7 is an exemplary diagram illustrating a needle guideline according to an embodiment of the present invention;

FIG. 8 is an exemplary diagram illustrating a first average value, a second average value, a start point, an end point, and a length for calculating an input angle of a needle, according to an embodiment of the present invention; and

FIG. 9 is an exemplary diagram illustrating a second region of interest (ROI), a plurality of angles, and a plurality of lines for calculating an input angle of a needle, according to an embodiment of the present invention.

DETAILED DESCRIPTION

All terms including descriptive or technical terms which are used herein should be construed as having meanings that are obvious to one of ordinary skill in the art. However, the terms may have different meanings according to an intention of one of ordinary skill in the art, precedent cases, or the appearance of new technologies. In addition, some terms may be arbitrarily selected by the applicant, and in this case, the meaning of the selected terms will be described in detail in the detailed description. Thus, the terms used herein should be defined based on the meaning of the terms together with the description throughout the specification.

Further, when a part "includes" or "comprises" an element, unless there is a particular description contrary thereto, the part can further include other elements, not excluding the other elements. In the following description, terms such as "unit" and "module" indicate a unit for processing at least one function or operation, wherein the unit and the block may be embodied as hardware or software or embodied by combining hardware and software.

Throughout the specification, "an ultrasound image" refers to an image which is obtained from an object by using ultrasonic waves. The object may refer to a part of the body. For example, the object may include an organ such as any one or more of a liver, a heart, a uterus, a brain, a breast, or an abdomen, or a fetus.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, embodiments of the present invention will be described in detail with reference to the accompanying drawings.

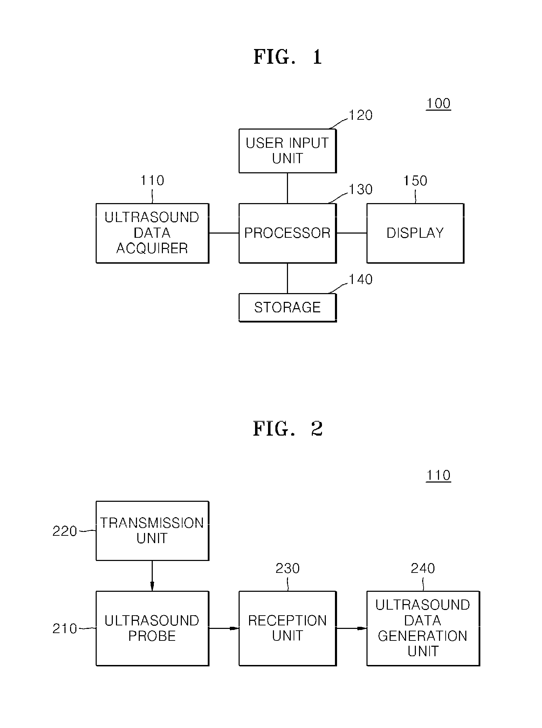

FIG. 1 is a block diagram of a configuration of an ultrasound system 100 according to an embodiment of the present invention. Referring to FIG. 1, the ultrasound system 100 includes an ultrasound data acquirer 110, a user input unit 120, a processor 130, a storage 140, and a display 150. Also, the ultrasound system 100 includes a medical instrument (not shown) that is inserted into an object in a freehand type. The medical instrument includes a needle. However, the medical instrument is not limited thereto.

The ultrasound data acquirer 110 transmits an ultrasound signal to an object. The object includes an object (for example, a lesion, a heart, a liver). Also, the ultrasound data acquirer 110 receives the ultrasound signal (namely, an ultrasound echo signal) reflected from the object to acquire ultrasound data corresponding to an ultrasound image.

FIG. 2 is a block diagram of a configuration of the ultrasound data acquirer 110 according to an embodiment of the present invention. Referring to FIG. 2, the ultrasound data acquirer 110 includes an ultrasound probe 210, a transmission unit 220, a reception unit 230, and an ultrasound data generation unit 240.

The ultrasound probe 210 includes a plurality of transducer elements (not shown) that convert between an electrical signal and an ultrasound signal. The ultrasound probe 210 transmits an ultrasound signal to an object, and receives an ultrasound echo signal reflected from the object to output an electrical signal (hereinafter, referred to as a reception signal). The reception signal is an analog signal. The ultrasound probe 210 includes a convex probe, a linear probe, and a 3D probe.

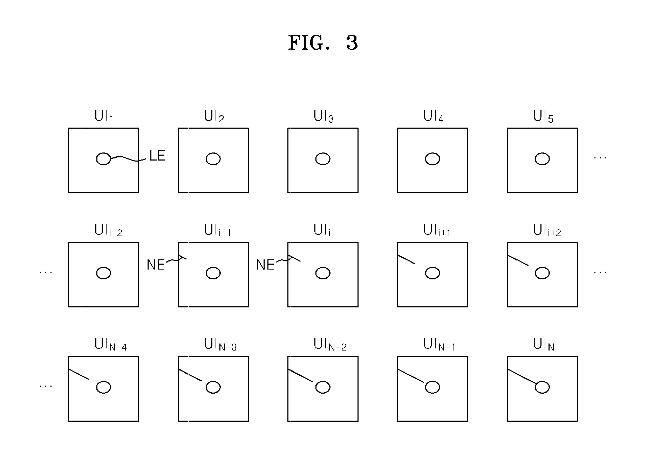

The transmission unit 220 controls transmission of an ultrasound signal. Also, the transmission unit 220 generates an electrical signal (hereinafter, referred to as a transmission signal) for obtaining an ultrasound signal in consideration of a transducer element and a focusing point. In the embodiment, as illustrated in FIG. 3, the transmission unit 220 sequentially generates a plurality of transmission signals for respectively obtaining a plurality of ultrasound images ($UI_N$ ($N \geq 1$)). In FIG. 3, reference numeral LE refers to an lesion, and reference numeral NE refers to a medical instrument, namely, a needle. Therefore, the ultrasound probe 210 converts each of the transmission signals, sequentially supplied from the transmission unit 220, into an ultrasound signal, transmits the ultrasound signal to an object, and receives an ultrasound echo signal reflected from the object to generate a reception signal.

The reception unit 230 analog-digital-converts the reception signal supplied from the ultrasound probe 210 to generate a digital signal. Also, the reception unit 230 performs reception beamforming on the digital signal in consideration of the transducer element and the focusing point to generate a reception focusing signal. Reception beamforming may use various known methods, and thus, a detailed description thereof is not provided in the embodiment. In the embodiment, the reception unit 230 analog-digital-converts each of a plurality of reception signals sequentially supplied from the ultrasound probe 210 to generate a digital signal, and performs reception beamforming on the digital signal in consideration of the transducer element and the focusing point to generate a reception focusing signal.

The ultrasound data generation unit 240 generates ultrasound data corresponding to an ultrasound image by using the reception focusing signal supplied from the reception unit 230. The ultrasound data includes radio frequency (RF) data. However, the ultrasound data is not limited thereto. In the embodiment, the ultrasound data generation unit 240 generates ultrasound data corresponding to each of a plurality of ultrasound images ($UI_N$ ($N \geq 1$) by using a plurality of reception focusing signals sequentially supplied from the reception unit 230. Also, the ultrasound data acquirer 110 may perform various signal processing (for example, gain adjustment, etc.), which is necessary to generate the ultrasound data, on the reception focusing signal.

Referring again to FIG. 1, the user input unit 120 receives a user's input information. In the embodiment, the input information includes point setting information for setting a point on an ultrasound image. That is, the point setting information includes a size and position information of a point which is set on a lesion of the ultrasound image. However, the input information is not limited thereto. The user input unit 120 includes at least one of a control panel, a trackball, a touch screen, a keyboard, and a mouse.

The processor 130 is connected to the ultrasound data acquirer 110 and the user input unit 120. The processor 130 includes a central processing unit (CPU), a microprocessor, or a graphics processing unit (GPU).

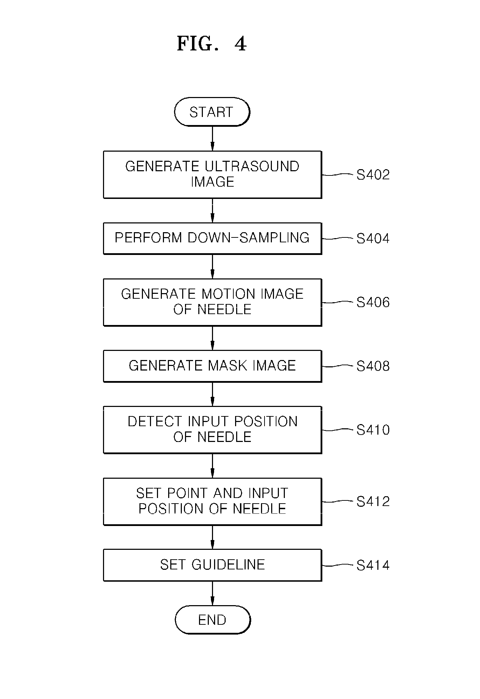

FIG. 4 is a flowchart of an operation of setting a guideline of a medical instrument (i.e., a needle), according to an embodiment of the present invention. Referring to FIG. 4, the processor 130 generates an ultrasound image ($UI_N$ ($N \geq 1$)) by using ultrasound data sequentially supplied from the ultrasound data acquirer 110 in operation S402.

The processor 130 down-samples the ultrasound image ($UI_N$ ($N \geq 1$)) to generate a down-sampled ultrasound image ($CUI_N$ ($N \geq 1$)) (hereinafter, referred to as a copy ultrasound image), for reducing a processing time and a storage space of the ultrasound image in operation S404. The down-sampling may use various known methods, and thus, a detailed description thereof is not provided in the embodiment.

The processor 130 generates a motion image (hereinafter, referred to as a needle motion image) by using a certain number of copy ultrasound images in operation S406. In the embodiment, the processor 130 generates a needle motion image by using a certain number (for example, five) of copy ultrasound images $CUI_{i-2}$, $CUI_{i-1}$, $CUI_i$, $CUI_{i+1}$ and $CUI_{i+2}$ in a temporal direction with respect to the copy ultrasound image $CUI_i$. For example, the processor 130 generates a needle motion image $NMI_i$ with the following Equation (1):

$$NMI_i = \frac{|CUI_{i-2}-CUI_{i-1}|+|CUI_{i-1}-CUI_i|+|CUI_i-CUI_{i+1}|+|CUI_{i+1}-CUI_{i+2}|}{4} \quad \cdots(1)$$

The processor 130 generates a mask image by using the ultrasound image $UI_i$ and the needle motion image $NMI_i$ in operation S408. The mask image is an image for emphasizing a needle portion in the ultrasound image $UI_i$. In the embodiment, the processor 130 generates a mask image $MI_i$ with the following Equation (2):

$$MI_i = UI_i \times \alpha + NMI_i \times (1-\alpha) \qquad \cdots(2)$$

where $\alpha$ denotes a weight value, and is a preset value or a value set by a user.

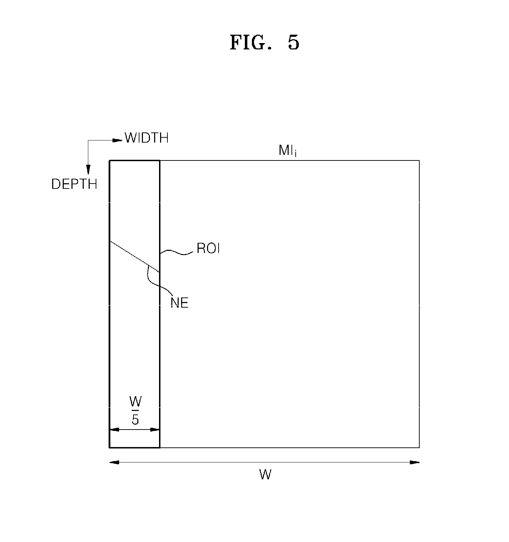

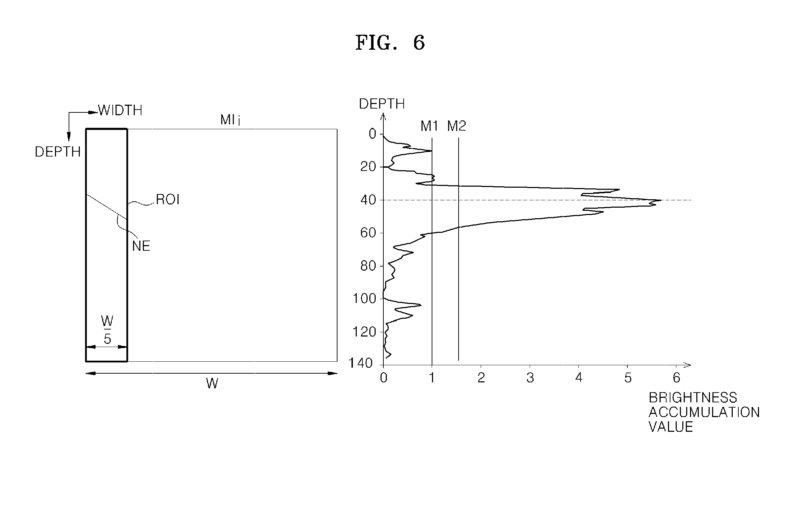

The processor 130 detects an input position (an insertion position) of a needle by using the mask image $MI_i$. In the embodiment, the processor 130 sets a region of interest (ROI) having a predetermine size in the mask image $MI_i$ in consideration of an input direction of the needle. The input direction of the needle may be manually set by a user, or may be automatically set by a system. For example, as illustrated in FIG. 5, the processor 150 sets an ROI having a size of a width W "0.2 × mask image $MI_i$" from the left of the mask image $MI_i$, in consideration of an input direction in which a needle NE is input from the left to the right with respect to the mask image $MI_i$. The processor 130, as illustrated in FIG. 6, accumulates brightness values of respective pixels having different depths in the ROI in a width direction of the mask image $MI_i$, thereby calculating a brightness accumulation value for each of the depths. The processor 130 detects the maximum brightness accumulation value from a plurality of the calculated brightness accumulation values, and detects a depth corresponding to the detected maximum brightness accumulation value as a needle input position NIP.

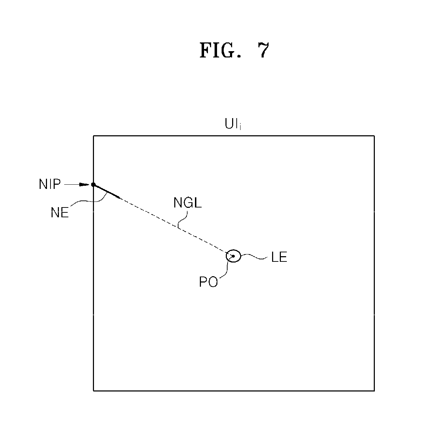

The processor 130, as illustrated in FIG. 7, sets the needle input position NIP and a point in the ultrasound image $UI_i$, on the basis of the needle input position NIP and input information (i.e., input information provided from the user input unit 120). The processor 130 sets a line, which connects the needle input position NIP and a point PO, as a needle guideline NGL.

In the above-described embodiment, it has been described above that an ultrasound image is down-sampled, and a needle motion image is generated by using the down-sampled image. However, in another embodiment, a needle motion image may be generated by using an ultrasound image without down-sampling the ultrasound image.

Alternatively, the processor 130 may perform motion tracking between adjacent ultrasound images with respect to a point set on each of a plurality of ultrasound images to detect a motion of the point, and reflect the detected motion of the point to set a guideline, indicating a path through which a needle is input, in an ultrasound image ($UI_N$ ($N{\geq}1$)).

Alternatively, the processor 130 may measure a distance between a point and an input position of a needle, and generate additional information including the measured distance. The distance may be measured by various known methods, and thus, a detailed description thereof is not provided in the embodiment.

Alternatively, as illustrated in FIG. 7, the processor 130 may calculate an angle (i.e., an angle indicating a degree of slope of a path through which a needle is input) of the guideline NGL with respect to the needle input position NIP, and generate additional information including the calculated angle. The angle may be calculated by various known methods, and thus, a detailed description thereof is not provided in the embodiment.

Alternatively, the processor 130 may calculate an input angle (an insertion angle) of a needle on the basis of the needle input position NIP, and generate additional information including the calculated input angle of the needle.

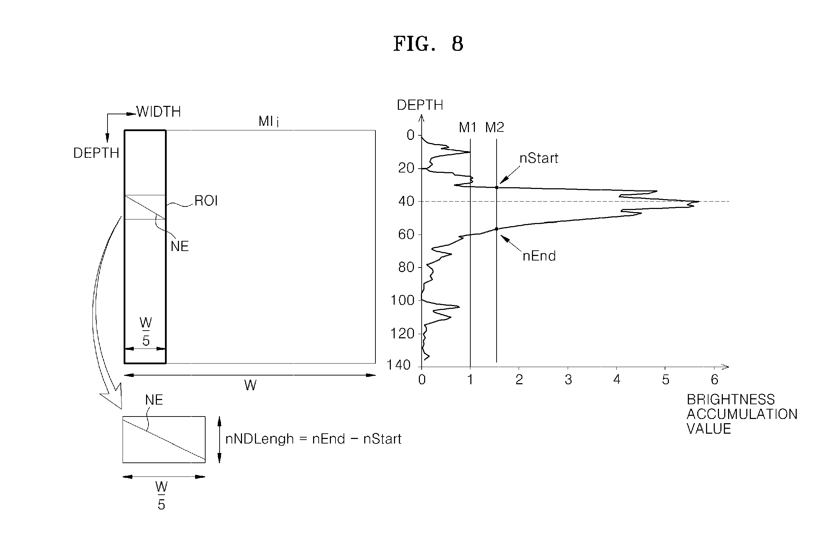

In an embodiment, as illustrated in FIG. 8, the processor 130 calculates a first average value "m1" of brightness accumulation values. The processor 130, as illustrated in FIG. 8, calculates a second average value "m2" of brightness accumulation values which are obtained by subtracting the first average value "m1" from the brightness accumulation values. The processor 130, as illustrated in FIG. 8, detects an intersection point between the second average value "m2" and the brightness accumulation values as a start point "nStart" and an end point "nEnd". The processor 130 calculates a length "nNDLength" between the detected start point "nStart" and end point "nEnd". The processor 130 calculates an input angle of a needle on the basis of the calculated length "nNDLength". For example, the processor 130 may calculate the input angle "$\Theta_{IA}$" of the needle with the following Equation (3);

$$\theta_{IA} = \tan^{-1}\left(\frac{nNDLength}{\frac{W}{5}}\right) \qquad \cdots(3)$$

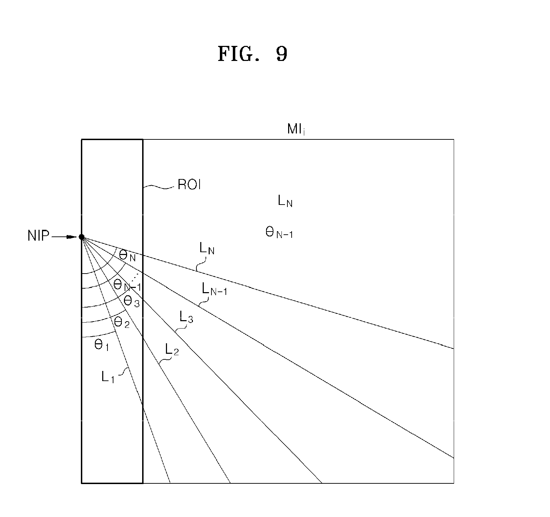

In another embodiment, as illustrated in FIG. 9, the processor 130 sets a plurality of angles "$\Theta_i$($1{\leq}i{\leq}N$)" with respect to the needle input position NIP, and sets a line "$L_i$($1{\leq}i{\leq}N$)" corresponding to each of the plurality of angles "$\Theta_i$($1{\leq}i{\leq}N$)" from the needle input position NIP. The processor 130 calculates brightness accumulation values of pixels respectively corresponding to the lines "$L_i$($1{\leq}i{\leq}N$)". The processor 130 detects the maximum brightness accumulation value from the calculated brightness accumulation values, and detects an angle, corresponding to the detected maximum brightness accumulation value, as an input angle of the needle.

Referring again to FIG. 1, the storage 140 stores ultrasound data acquired by the ultrasound data acquirer 110. Also, the storage 140 stores input information received by the user input unit 120. Also, the storage 140 stores an ultrasound image and a copy ultrasound image, which are generated by the processor 130. Also, the storage 140 stores additional information (including an input position, input angle, and guideline angle of a needle) generated by the processor 130.

The display 150 displays the ultrasound image and/or copy ultrasound image which are/is generated by the processor 130. Also, the display 150 displays the guideline set by the processor 130. Also, the display 150 displays the additional information (including the input position, input angle, and guideline angle of a needle) generated by the processor 130.

As described above, an ultrasound system provides a guideline of a needle in the freehand type that does not use an additional device such as a sensor and the like, and thus informs a user of a directionality and quantitative numerical information such as display of an angle and a distance. Accordingly, an accuracy of an interventional procedure using a needle increases, and a time is shortened in the interventional procedure.

It should be understood that the exemplary embodiments described therein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

**Claims**

1. An ultrasound system comprising:

   an ultrasound data acquirer that transmits an ultrasound signal to an object with a needle inserted thereinto, and acquires ultrasound data corresponding to each of a plurality of ultrasound images by receiving an ultrasound echo signal reflected from the object;
   a user input unit that receives input information, used to set a point in an ultrasound image, from a user; and
   a processor that generates the plurality of ultrasound images with the ultrasound data, generates a mask image used to detect an input position of the needle by using the plurality of ultrasound images, detects the input position by using the mask image, and sets a guideline of the needle in the ultrasound image on a basis of the input position and the input information.

2. The ultrasound system of claim 1, wherein the processor generates a plurality of copy ultrasound images by down-sampling the plurality of ultrasound images.

3. The ultrasound system of claim 2, wherein the processor generates a motion image of the needle by using a certain number of copy ultrasound images among the plurality of copy ultrasound images.

4. The ultrasound system of claim 3, wherein the processor selects a certain number of copy ultrasound images in a temporal direction with respect to each of the plurality of copy ultrasound images.

5. The ultrasound system of claim 4, wherein the processor generates the motion image of the needle with a following Equation (1):

$$NMI_i = \frac{|CUI_{i-2} - CUI_{i-1}| + |CUI_{i-1} - CUI_i| + |CUI_i - CUI_{i+1}| + |CUI_{i+1} - CUI_{i+2}|}{4} \qquad \cdots (1)$$

where $NMI_i$ denotes a needle motion image, and $CUI_{i-2}$ to $CUI_{i+2}$ respectively denote the selected copy ultrasound images.

6. The ultrasound system of claim 3, wherein the processor generates a mask image used to emphasize the needle in the ultrasound image by using the ultrasound image and the motion image.

7. The ultrasound system of claim 6, wherein the processor sets a region of interest (ROI) having a predetermined size in the mask image in consideration of an input direction of the needle, calculate a plurality of brightness accumulation values by accumulating brightness values of respective pixels having different depths of the mask image in a width direction of the mask image, for a plurality of pixels in the ROI, detects a maximum brightness accumulation value from the plurality of the calculated brightness accumulation values, and detects a depth, corresponding to the detected maximum brightness accumulation value, as the input position of the needle.

8. The ultrasound system of claim 7, wherein the processor sets the input position and the point in the ultrasound image based on the input position of the needle and the input information, and sets a line, which connects the input position and the point, as the guideline.

9. The ultrasound system of claim 8, wherein the processor detects a motion of the point by performing motion tracking between adjacent ultrasound images with respect to the point set on each of the plurality of ultrasound images.

10. The ultrasound system of claim 8, wherein the processor measures a distance between the point and the input position.

11. The ultrasound system of claim 8, wherein the processor calculates an angle of the guideline with respect to the input position.

12. The ultrasound system of claim 8, wherein the processor detects an input angle of the needle on a basis of the input position.

13. The ultrasound system of claim 12, wherein the processor calculates a first average value of the plurality of brightness accumulation values, calculates a second average value of brightness accumulation values which are obtained by subtracting the first average value from the plurality of the brightness accumulation values, detects an intersection point between the second average value and the plurality of brightness accumulation values as a start point and an end point, calculates a length between the detected start point and end point, and calculates the input angle of the needle based on the calculated length.

14. The ultrasound system of claim 12, wherein the processor sets a plurality of angles with respect to the input position of the needle, sets a line corresponding to each of the plurality of angles from the input position of the needle, calculates brightness accumulation values of pixels of a line corresponding to each of the plurality of angles, calculates a maximum brightness accumulation value from the calculated brightness accumulation values, and detects an angle, corresponding to the detected maximum brightness accumulation value, as the input angle of the needle.

15. A method of providing a guideline of a needle, the method comprising:

a) transmitting an ultrasound signal to an object with a needle inserted thereinto, and acquires ultrasound data corresponding to each of a plurality of ultrasound images by receiving an ultrasound echo signal reflected from the object;
b) generating the plurality of ultrasound images with the ultrasound data;
c) receiving input information, used to set a point in an ultrasound image, from a user;
d) generating a mask image used to detect an input position of the needle by using the plurality of ultrasound images;
e) detecting the input position by using the mask image; and
f) setting a guideline of the needle in the ultrasound image based on the input position and the input information.

FIG. 1

<u>100</u>

```
                    ┌─────────────┐ 120
                    │ USER INPUT  │
                    │    UNIT     │
                    └──────┬──────┘
 110                       │              150
┌─────────────┐    ┌──────┴──────┐ 130  ┌─────────────┐
│ ULTRASOUND  │    │             │      │             │
│    DATA     ├────┤  PROCESSOR  ├──────┤   DISPLAY   │
│  ACQUIRER   │    │             │      │             │
└─────────────┘    └──────┬──────┘      └─────────────┘
                          │
                   ┌──────┴──────┐ 140
                   │   STORAGE   │
                   └─────────────┘
```

FIG. 2

<u>110</u>

```
        ┌──────────────┐
        │ TRANSMISSION │
 220────┤     UNIT     │
        └──────┬───────┘
               │
               ▼              230              240
        ┌──────────────┐ ┌──────────────┐ ┌──────────────┐
        │  ULTRASOUND  │ │  RECEPTION   │ │  ULTRASOUND  │
 210────┤    PROBE     ├─┤     UNIT     ├─┤     DATA     │
        │              │ │              │ │  GENERATION  │
        └──────────────┘ └──────────────┘ │     UNIT     │
                                          └──────────────┘
```

FIG. 3

# FIG. 4

START

GENERATE ULTRASOUND IMAGE — S402

PERFORM DOWN-SAMPLING — S404

GENERATE MOTION IMAGE OF NEEDLE — S406

GENERATE MASK IMAGE — S408

DETECT INPUT POSITION OF NEEDLE — S410

SET POINT AND INPUT POSITION OF NEEDLE — S412

SET GUIDELINE — S414

END

FIG. 5

## FIG. 6

EP 2 735 271 A1

FIG. 7

FIG. 8

EP 2 735 271 A1

FIG. 9

Europäisches
Patentamt

European
Patent Office

Office européen
des brevets

# EUROPEAN SEARCH REPORT

Application Number

EP 13 19 1054

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/249878 A1 (PAGOULATOS NIKOLAOS [US] ET AL) 13 October 2011 (2011-10-13) * abstract * * figures 1-8 * * paragraph [0028] - paragraph [0058] * | 1-15 | INV. A61B8/08 A61B8/00 A61B19/00 G06T7/00 |
| X | US 2011/201931 A1 (PALMERI MARK L [US] ET AL) 18 August 2011 (2011-08-18) * abstract * * figures 1-22 * * paragraph [0049] - paragraph [0104] * | 1,15 | |
| A | US 2009/093717 A1 (CARNEIRO GUSTAVO HENRIQUE MONT [PT] ET AL MONTEIRO DE BARROS CARNEIRO) 9 April 2009 (2009-04-09) * abstract * * paragraph [0032] * * paragraph [0044] * | 2-14 | |
| A | US 2012/253181 A1 (OKAMURA YOKO [JP] ET AL) 4 October 2012 (2012-10-04) * abstract * * figures 1-14 * * paragraph [0021] - paragraph [0146] * | 1-15 | TECHNICAL FIELDS SEARCHED (IPC) A61B G06T |

The present search report has been drawn up for all claims

| Place of search | Date of completion of the search | Examiner |
|---|---|---|
| Munich | 20 December 2013 | Moehrs, Sascha |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 13 19 1054

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2013

| Patent document cited in search report | | Publication date | Patent family member(s) | | Publication date |
|---|---|---|---|---|---|
| US 2011249878 | A1 | 13-10-2011 | CA<br>CN<br>EP<br>JP<br>US<br>WO | 2796067 A1<br>103237499 A<br>2555683 A1<br>2013523343 A<br>2011249878 A1<br>2011127191 A1 | 13-10-2011<br>07-08-2013<br>13-02-2013<br>17-06-2013<br>13-10-2011<br>13-10-2011 |
| US 2011201931 | A1 | 18-08-2011 | NONE | | |
| US 2009093717 | A1 | 09-04-2009 | NONE | | |
| US 2012253181 | A1 | 04-10-2012 | CN<br>JP<br>US | 102727250 A<br>2012213606 A<br>2012253181 A1 | 17-10-2012<br>08-11-2012<br>04-10-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020120134005